# EUROPEAN PATENT APPLICATION

(11) **EP 3 865 111 A1**
(43) Date of publication of application: **18.08.2021**
(21) Application number: 19742514.3
(22) Date of filing: 13.05.2019
(51) Int. Cl.: A61K 8/02, A61Q 19/00

(54) **FACIAL MASK SHEET, FACIAL MASK ASSEMBLY, AND METHOD FOR MANUFACTURING SAME**

(30) Priority: 14.09.2018 CN 201811072208
(71) Applicant: Yangshengtang (ANJI) Cosmetics Co., Ltd., Huzhou, Zhejiang 313399 (CN)
(72) Inventor: ZHAO, Shizhi, Huzhou Zhejiang 313399 (CN); YANG, Shan, Huzhou Zhejiang 313399 (CN)
(74) Representative: J A Kemp LLP
(86) International application number: PCT/CN2019/086651
(87) International publication number: WO 2020/052250

(57) **Abstract**

The present disclosure relates to a facial mask sheet, a facial mask component and a manufacturing process of facial mask thereof, and relates to the field of cosmetics package. The structure of the existing facial mask sheet is optimized. The facial mask sheet includes a main body and protrusions. The main body is provided with eye holes and a mouth hole, and the eye holes and the mouth hole are symmetrically arranged with respect to a vertical center line of the main body. A plurality of protrusions are arranged along the edge of the main body, and the plurality of protrusions are asymmetrically arranged with respect to the vertical center line. With respect to the facial mask sheet with the above structure, the facial mask sheet can be easily unfolded, thus improving the convenience of use, and meanwhile avoiding the migration of potentially harmful substances such as heavy metals, fluorescent agents, pigments and the like onto the mask cloth, resulting in harm to the skin when the facial mask is used.

## Description

The present application claims the priority of Chinese Application No. 201811072208.9, filed in the Chinese Patent Office on September 14, 2018, and the entire contents of which are herein incorporated by reference.

### Field of the Invention

The present disclosure relates to the field of cosmetic packaging, and in particular to a facial mask sheet, a facial mask component and a manufacturing process of facial mask.

### Description of Related Art

At present, thin facial masks with high skin fitting degrees on the market have very thin mask cloth, once being immersed in essence liquid, the mask cloth is adhered together and is difficult to be separated after absorbing the essence. Even if the mask cloth is separated, the mask cloth is easily pulled and deformed in the separation process, affecting the use of the facial masks.

In order to support the support mask cloth and facilitate the separation of the mask cloth, blue films, pearlized films and the like are used as the linings of the thin facial masks in related art.

The inventors have found that at least the following problems exist in the related art: the blue films, the pearlized films and the like are prone to problems such as excessive heavy metals, fluorescent agent exudation, pigment migration and the like, which are potentially harmful to the human body.

### Summary of the Invention

According to one aspect of some embodiments of the present disclosure, the embodiment of the present disclosure provides a facial mask sheet, including:
a main body, provided with eye holes and a mouth hole, wherein the eye holes and the mouth hole are symmetrically arranged with respect to a vertical center line of the main body; and
a plurality of protrusions, arranged along the edge of the main body, and the protrusions are asymmetrically arranged with respect to the vertical center line of the main body.

In some embodiments, the protrusions and the main body are integrated.

In some embodiments, the protrusions are in smooth transition with the main body.

In some embodiments, the protrusion is semi-circular.

In some embodiments, a plurality of protrusions comprise at least two protrusions; two protrusions are arranged on the edge of the main body and are higher than the positions of the eye holes, and the two protrusions are arranged on the two sides of the eye holes, wherein when the facial mask sheet is folded in half along the vertical center line of the main body, the two protrusions are staggered.

Some other embodiments of the present disclosure provide a facial mask component, comprising a packaging bag and the facial mask sheet provided by any one of the technical solutions of the present disclosure, wherein the facial mask sheet in the packaging bag is folded.

In some embodiments, the facial mask sheet is configured to be folded in half twice along the vertical direction and folded in half once along the horizontal direction, or the facial mask sheet is configured to be folded in half once along the vertical direction and folded in half twice along the horizontal direction; or the facial mask sheet is folded in half once along the vertical direction and is horizontally folded once along a horizontal center line on which the eye holes are located and a horizontal center line on which the lower edge of the mouth hole is located respectively;
the vertical direction is parallel or overlapped with a vertical center line of the main body; and the horizontal direction is located in a plane where the facial mask sheet is located and is perpendicular to the vertical direction.

In some embodiments, the facial mask sheet is configured to be folded in the following order: the facial mask sheet is folded in half once along the vertical center line of the facial mask sheet to obtain a first folded body; the first folded body is folded in half once along the vertical center line of the first folded body to obtain a second folded body; and the second folded body is folded along the horizontal center line of the second folded body to obtain a folded facial mask.

In some embodiments, the facial mask sheet is configured to be folded in the following order: the facial mask sheet is folded in half once along the vertical center line of the facial mask sheet to obtain the first folded body; the first folded body is folded in half once along the horizontal center line of the first folded body to obtain the second folded body; and the second folded body is folded along the vertical center line of the second folded body to obtain the folded facial mask.

In some embodiments, the facial mask sheet is configured to be folded in the following order: the facial mask sheet is folded in half once along the vertical center line of the facial mask sheet to obtain the first folded body; the first folded body is folded in half once along the horizontal center line of the first folded body to obtain the second folded body; and the second folded body is folded along the horizontal center line of the second folded body to obtain the folded facial mask.

In some embodiments, the facial mask sheet is configured to be folded in the following order:
the facial mask sheet is folded in half once along the vertical center line of the facial mask sheet to obtain the first folded body;
the first folded body is folded once from top to bottom along the horizontal center line on which the eye hole of the first folded body is located, so as to obtain the second folded body; and
the second folded body is folded from bottom to top along the horizontal line on which the lower edge of the mouth hole of the second folded body is located, so as to obtain the folded facial mask.

In some embodiments, the facial mask component has no isolating membrane.

Some other embodiments of the present disclosure provide a manufacturing process of facial mask, including the following steps:
folding a facial mask sheet according to any one of the technical solutions of the present disclosure in half twice along the vertical direction and folding the same mask sheet in half once along the horizontal direction to obtain a folded facial mask, or folding the facial mask sheet in half once along the vertical direction and folding the same mask sheet in half twice along the horizontal direction to obtain the folded facial mask; or folding the facial mask sheet in half once along the vertical direction and horizontally folding the same mask sheet once along horizontal center lines on which the eye holes are located and the horizontal line on which a lower edge of the mouth hole is located respectively to obtain the folded facial mask; and
placing the folded facial mask in a packaging bag.

In some embodiments, folding a facial mask sheet in half twice along the vertical direction and folding the same mask sheet in half once along the horizontal direction to obtain a folded facial mask includes the following steps:
folding the facial mask sheet in half once along the vertical center line of the facial mask sheet to obtain a first folded body;
folding the first folded body in half once along the vertical center line of the first folded body to obtain a second folded body; and
folding the second folded body along the horizontal center line of the second folded body to obtain the folded facial mask.

In some embodiments, folding a facial mask sheet in half twice along the vertical direction and folding the same mask sheet in half once along the horizontal direction to obtain a folded facial mask includes the following steps:
folding the facial mask sheet in half once along the vertical center line of the facial mask sheet to obtain the first folded body;
folding the first folded body in half once along the horizontal center line of the first folded body to obtain the second folded body; and
folding the second folded body along the vertical center line of the second folded body to obtain the folded facial mask.

In some embodiments, folding the facial mask sheet in half once along the vertical direction and folding the same mask sheet in half twice along the horizontal direction to obtain the folded facial mask includes the following steps:
folding the facial mask sheet in half once along the vertical center line of the facial mask sheet to obtain the first folded body;
folding the first folded body in half once along the horizontal center line of the first folded body to obtain the second folded body; and
folding the second folded body along the horizontal center line of the second folded body to obtain the folded facial mask.

In some embodiments, folding the facial mask sheet in half once along the vertical direction and horizontally folding the same mask sheet once along horizontal center lines of the eye holes and the horizontal line on which a lower edge of the mouth hole is located respectively to obtain the folded facial mask includes the following steps:
folding the facial mask sheet in half once along the vertical center line of the facial mask sheet to obtain the first folded body;
folding the first folded body once from top to bottom along the horizontal center line of the eye hole of the first folded body to obtain the second folded body; and
folding the second folded body from bottom to top along the horizontal line on which the lower edge of the mouth hole of the second folded body is located to obtain the folded facial mask.

In some embodiments, the manufacturing process of facial mask further includes the following steps:
filling the packaging bag with fluid; and
encapsulating the packaging bag.

The facial mask sheet provided by the above technical solutions is provided with the protrusions, and the protrusions are asymmetrically arranged along the vertical center line of the main body. When the facial mask is manufactured, the facial mask sheet is folded into the folded facial mask to be placed into the packaging bag. After the facial mask sheet with the above structure is folded, the protrusions protrude from the contour of the main body, so that consumers easily open the facial mask during use, and the deformation of the facial mask caused by pulling is reduced or even prevented. Even if the facial mask sheet adopts the existing thin mask, the facial mask sheet is easily unfolded without providing the isolating membrane, thus improving the convenience of use.

### Brief Description of the Drawings

Fig.1 is a structural schematic diagram of a facial mask sheet provided by some embodiments of the present disclosure.
Fig.2 is a schematic flow diagram of a manufacturing process of facial mask provided by some embodiments of the present disclosure.
Fig.3a to Fig.3e are schematic diagrams of a process of folding a facial mask sheet.

### Description of the Embodiments

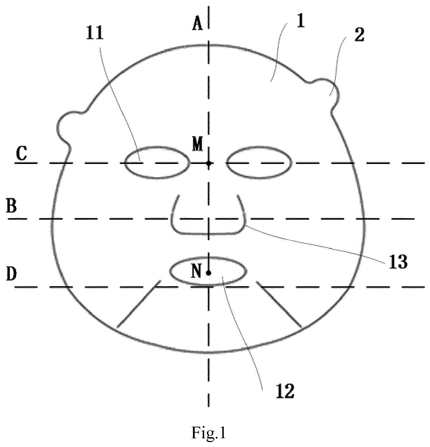

The technical solutions provided by the present disclosure will be described in more detail below with reference to Fig.1 to Fig.3e.

The orientations used herein are explained. Taking it as an example that a facial mask sheet is tiled, the direction of a connecting line A of the midpoint of an eye hole 11 and a mouth hole 12 is a vertical direction. A vertical center line A is the vertical center line of a main body 1 and is also the vertical center line of the facial mask sheet. In a plane of the facial mask sheet, the direction of a line B vertical to the line A is a horizontal direction.

Referring to Fig.1, the embodiments of the present disclosure provides a facial mask sheet, including a main body 1 and protrusions 2. The main body 1 is provided with eye holes 11 and a mouth hole 12. The eye holes 11 and the mouth hole 12 are symmetrically arranged with respect to the vertical center line A of the main body 1. A plurality of protrusions 2 are arranged along the edge of the main body 1. The plurality of protrusions 2 are asymmetrically arranged with respect to the vertical center line A of the main body 1.

The so-called asymmetric arrangement means that after the main body 1 is folded in half along the vertical center line A, the protrusions 2 are all or partially staggered. The partial stagger herein means that a part of protrusions 2 are staggered to each other, or only a part of area of one protrusion 2 is staggered to a part of the other protrusion 2.

The number of the protrusions 2 is two or more, and in some embodiments, two are exemplified.

Referring to Fig.1, in some embodiments, two protrusions 2 are arranged on the edge of the main body 1 and are higher than the positions of the eye holes 11, and the two protrusions 2 are arranged on the two sides of the eye holes 11. When the facial mask sheet is in a state of being folded in half along the vertical center line A of the main body 1, and the two protrusions 2 are staggered. The vertical center line A of the facial mask sheet is a connecting line between midpoints M of inner edges of the two eye holes 11 and the midpoint N of the mouth hole 12. The vertical center line of the facial mask sheet is overlapped with the vertical center line of the main body 1.

The size of the protrusion 2 is suitable for pinching a thumb and a forefinger.

Referring to Fig.1, taking the left-right direction shown in Fig.1 as an example, the position of the protrusion 2 on one side is completely higher than or partially higher than the position of the protrusion 2 on the other side. Specifically, for example, the position of the protrusion 2 located on the left side is high, and the position of the protrusion 2 located on the right side is low. Or, the position of the protrusion 2 on the left side is low, and the position of the protrusion 2 on the right side is high.

Referring to Fig.1, in some embodiments, the protrusion 2 and the main body 1 are integrated. For example, the protrusion 2 and the main body 1 are integrally cut.

Referring to Fig.1, in some embodiments, the protrusion 2 is in smooth transition with the main body 1. The smooth transition structure makes it difficult for the facial mask sheet to be torn from the connection site of the protrusion 2 and the main body 1, thereby product is reliable.

Referring to Fig.1, in some embodiments, the protrusion 2 is semi-circular, so that the structure can be conveniently formed.

Some other embodiments of the present disclosure provide a facial mask component, including a packaging bag and a facial mask sheet provided by any one of the technical solutions of the present disclosure, wherein the facial mask sheet is located in the packaging bag; and the facial mask sheet is in a folded state, that is, the facial mask sheet is folded into a folded facial mask, and the folded facial mask is placed in the packaging bag.

How to perform folding to form the folded facial mask is described below.

In some embodiments, the facial mask sheet is configured to be folded in half twice along the vertical direction and is folded in half once along the horizontal direction, or, the facial mask sheet is configured to be folded in half once along the vertical direction and is folded in half twice along the horizontal direction. The vertical direction refers to a direction wherein a connecting line of the midpoint M of the inner edges of the two eye holes 11 and the midpoint N of the mouth hole 12 is located, when the facial mask sheet is tiled, that is, the direction where the line A is located or the direction parallel to the line A in the plane of the facial mask sheet. The horizontal direction refers to a direction located in the plane where the facial mask sheet is located and perpendicular to the vertical direction, that is, the direction where the line B is located or the direction parallel to the line B in the plane of the facial mask sheet.

Herein, the folding in half refers to folding along a center line basically. Vertical folding in half refers to folding along the vertical center line of the body to be folded. Horizontal folding refers to folding along the horizontal center line of the body to be folded.

In some embodiments, the facial mask sheet is configured to be folded in the following order: the facial mask sheet is firstly folded in half once along the vertical center line of the facial mask sheet to obtain a first folded body. The first folded body is folded in half once along the vertical center line of the first folded body to obtain a second folded body. Finally, the second folded body is folded along the horizontal center line of the second folded body to obtain a folded facial mask. The obtained folded facial mask is placed in a packaging bag.

In some embodiments, the facial mask sheet is configured to be folded in the following order: the facial mask sheet is firstly folded in half once along the vertical center line of the facial mask sheet to obtain the first folded body, then the first folded body is folded in half once along the horizontal center line of the first folded body to obtain the second folded body, and finally the second folded body is folded along the vertical center line of the second folded body to obtain the folded facial mask. The obtained folded facial mask is placed in the packaging bag.

In some embodiments, the facial mask sheet is configured to be folded in the following order: the facial mask sheet is firstly folded in half once along the vertical center line of the facial mask sheet to obtain the first folded body, then the first folded body is folded in half once along the horizontal center line of the first folded body to obtain the second folded body, and finally the second folded body is folded along the horizontal center line of the second folded body to obtain the folded facial mask. The obtained folded facial mask is placed in the packaging bag.

In some embodiments, the facial mask sheet is configured to be folded in the following order: the facial mask sheet is folded in half once along the vertical center line of the facial mask sheet to obtain the first folded body. The first folded body is folded once from top to bottom along the horizontal center line C of the eye hole 11 of the first folded body to obtain the second folded body. The second folded body is folded from bottom to top along the horizontal line D on which the lower edge of the mouth hole 12 of the second folded body is located to obtain the folded facial mask. The obtained folded facial mask is placed in the packaging bag.

In some embodiments, the facial mask component has no isolating membrane, thereby avoiding the migration of potentially harmful substances such as heavy metals, fluorescent agents, pigments and the like onto the mask cloth, when the facial mask is used.

The embodiment of the present disclosure further provides a manufacturing process of facial mask for obtaining the facial mask component described in the above embodiment. The manufacturing process of facial mask includes the following steps:

Step S10. Folding a facial mask sheet in half twice along the vertical direction and folding the same mask sheet in half once along the horizontal direction to obtain a folded facial mask. Or, folding the facial mask sheet in half once along the vertical direction and folding the same mask sheet in half twice along the horizontal direction to obtain the folded facial mask. Or, folding the facial mask sheet in half once along the vertical center line A and horizontally folding the same mask sheet once along the horizontal center line C of the eye holes 11 and the horizontal line D on which a lower edge of the mouth hole 12 is located respectively to obtain the folded facial mask.

The facial mask sheet, for example, adopts the structure provided by any one of the above embodiments.

Step S20. Placing the folded facial mask in a packaging bag.

In some embodiments, the manufacturing process of facial mask further includes the following steps:

Step S30. Filling the packaging bag with fluid. For example, the fluid is essence liquid or other liquid cosmetics.

Step S40. Encapsulating the packaging bag.

It should be noted that the order of the step S30, the step S10 and the step S20 is not limited. In some embodiments, it is taken as an example that the facial mask sheet is firstly folded to obtain the folded facial mask, the folded facial mask is placed in the packaging bag, and then the packaging bag is filled with the liquid. It is understood that, the packaging bag is filled with the liquid at first, and then the folded facial mask is placed in the packaging bag.

Two manners of folding the facial mask sheet in half twice along the vertical direction and folding the same mask sheet in half once along the horizontal direction to obtain the folded facial mask are introduced below.

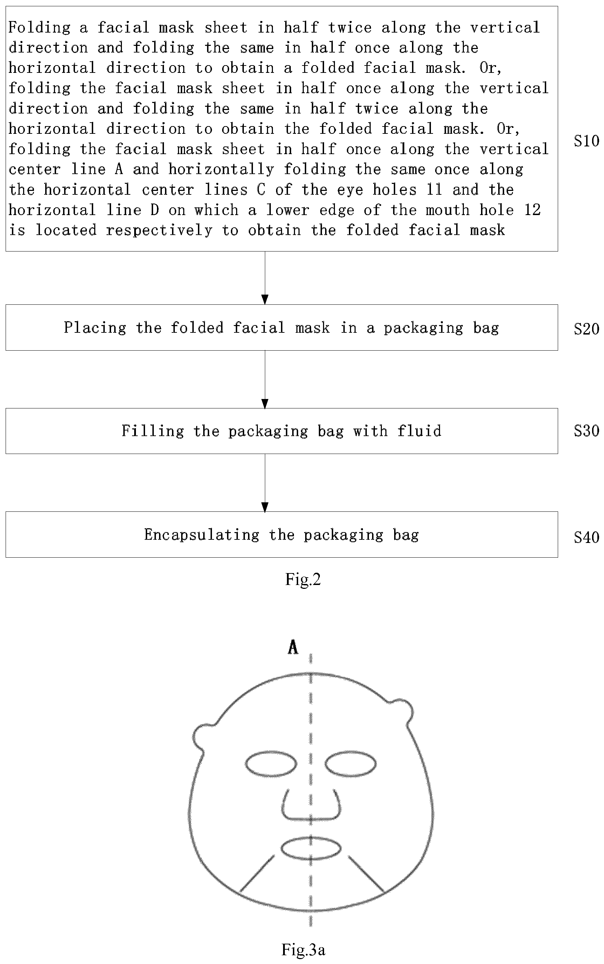

The first folding method is as follows, as shown in Fig.3a to Fig.3e:

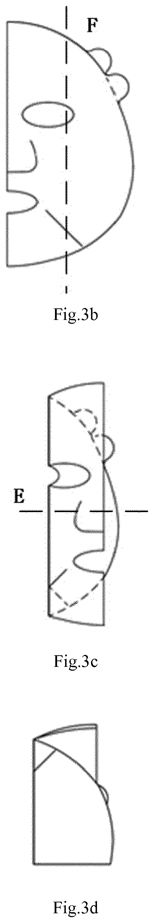

Firstly, the facial mask sheet is folded in half once along the vertical center line A of the facial mask sheet to obtain a first folded body, as shown in Fig.3a and Fig.3b.

Secondly, the first folded body is folded in half once along the vertical center line F of the first folded body to obtain a second folded body, as shown in Fig.3c.

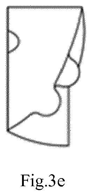

Finally, the second folded body is folded along the horizontal center line E of the second folded body to obtain the folded facial mask. Here, if the second folded body is folded from bottom to top, then the folded facial mask obtained by folding needs to be reversed, so that the protrusions 2 are located above. The manner is as shown in Fig.3d and Fig.3e. Of course, if the second folded body is directly folded from top to bottom, the state of Fig.3e can be directly obtained from the state as shown in Fig.3c.

The second folding method is as follows:

Firstly, the facial mask sheet is folded in half once along the vertical center line A of the facial mask sheet to obtain the first folded body.

Then, the first folded body is folded in half once along the horizontal center line of the first folded body to obtain the second folded body.

Finally, the second folded body is folded along the vertical center line of the second folded body to obtain the folded facial mask.

The specific manner of folding the facial mask sheet in half once along the vertical direction and folding the same in half twice along the horizontal direction to obtain the folded facial mask is introduced below.

Firstly, the facial mask sheet is folded in half once along the vertical center line of the facial mask sheet to obtain the first folded body.

Secondly, the first folded body is folded in half once along the horizontal center line of the first folded body to obtain the second folded body.

Finally, the second folded body is folded along the horizontal center line of the second folded body to obtain the folded facial mask.

Two detailed process of folding the facial mask sheet in half once along the vertical direction and horizontally folding the same mask sheet once along the horizontal center lines of the eye holes and the horizontal line on which the lower edge of the mouth hole is located respectively to obtain the folded facial mask are introduced below:

The first folding method is as follows:

Firstly, the facial mask sheet is folded in half once along the vertical center line A of the facial mask sheet to obtain the first folded body.

Secondly, the first folded body is folded once from top to bottom along the horizontal center line C of the eye hole 11 of the first folded body to obtain the second folded body.

Finally, the second folded body is folded from bottom to top along the horizontal line D on which the lower edge of the mouth hole 12 of the second folded body is located to obtain the folded facial mask.

The second folding method is as follows:

Firstly, the facial mask sheet is folded in half once along the vertical center line A of the facial mask sheet to obtain the first folded body.

Secondly, the first folded body is folded from bottom to top along the horizontal line D on which the lower edge of the mouth hole 12 is located to obtain the second folded body.

Finally, the second folded body is folded once from top to bottom along the horizontal center line C of the eye hole 11 to obtain the folded facial mask.

It should be noted that, with respect to the folded facial mask placed in the packaging bag, preferably the protrusions 2 are close to the front face of the packaging bag for ease of use.

In the description of the present disclosure, it should be understood that orientation or position relationships indicated by the terms "center", "longitudinal", "transverse", "front", "back", "left", "right", "vertical", "horizontal" and the like are orientation or position relationships shown in the drawings, are merely for the convenience of describing the present disclosure and simplifying the description, rather than indicating or implying that the devices or components referred to must have particular orientations, are constructed and operated in particular orientations, and thus cannot be construed as limiting the protection scope of the present disclosure.

Finally, it should be noted that the above embodiments are only used for illustrating the technical solutions of the present disclosure, rather than limiting the same; although the present disclosure has been described in detail with reference to the preferred embodiments, those of ordinary skill in the art to which the present disclosure belongs should understand that modifications can still be made to the specific embodiments of the present disclosure or equivalent substitutions are made to a part of technical features, without departing from the spirit of the technical solutions of the present disclosure, and these modifications and equivalent substitutions should be included in the scope of the technical solutions claimed in the present disclosure.

## Claims

1. A facial mask sheet, comprising:
a main body (1), provided with eye holes (11) and a mouth hole (12), wherein the eye holes (11) and the mouth hole (12) are symmetrically arranged with respect to a vertical center line of the main body (1); and
a plurality of protrusions (2), arranged along the edge of the main body (1), and the protrusions (2) are asymmetrically arranged with respect to the vertical center line of the main body (1).

2. The facial mask sheet according to claim 1, wherein the protrusions (2) and the main body (1) are integrated.

3. The facial mask sheet according to claim 1, wherein the protrusions (2) are in smooth transition with the main body (1).

4. The facial mask sheet according to claim 1, wherein the protrusions (2) are semi-circular.

5. The facial mask sheet according to claim 1, wherein a plurality of protrusions (2) comprise at least two protrusions (2); two protrusions (2) are arranged on the edge of the main body (1) and are higher than the positions of the eye holes (11), and the two protrusions (2) are arranged on the two sides of the eye holes (11), wherein when the facial mask sheet is folded in half along the vertical center line of the main body (1), the two protrusions (2) are staggered.

6. A facial mask component, comprising a packaging bag and the facial mask sheet according to any one of claims 1-5, wherein the facial mask sheet in the packaging bag is folded.

7. The facial mask component according to claim 6, wherein the facial mask sheet is configured to be folded in half twice along the vertical direction and folded in half once along the horizontal direction, or the facial mask sheet is configured to be folded in half once along the vertical direction and folded in half twice along the horizontal direction; or the facial mask sheet is folded in half once along the vertical direction and is horizontally folded once along a horizontal center line on which the eye holes (11) are located and a horizontal center line on which the lower edge of the mouth hole (12) is located respectively;
wherein the vertical direction is parallel or overlapped with a vertical center line of the main body (1); and the horizontal direction is in a plane in which the facial mask sheet is located and is perpendicular to the vertical direction.

8. The facial mask component according to claim 7, wherein the facial mask sheet is configured to be folded in the following order:
the facial mask sheet is folded in half once along the vertical center line of the facial mask sheet to obtain a first folded body;
the first folded body is folded in half once along the vertical center line of the first folded body to obtain a second folded body; and
the second folded body is folded along the horizontal center line of the second folded body to obtain a folded facial mask.

9. The facial mask component according to claim 7, wherein the facial mask sheet is configured to be folded in the following order:
the facial mask sheet is folded in half once along the vertical center line of the facial mask sheet to obtain the first folded body;
the first folded body is folded in half once along the horizontal center line of the first folded body to obtain the second folded body; and
the second folded body is folded along the vertical center line of the second folded body to obtain the folded facial mask.

10. The facial mask component according to claim 7, wherein the facial mask sheet is configured to be folded in the following order:
the facial mask sheet is folded in half once along the vertical center line of the facial mask sheet to obtain the first folded body;
the first folded body is folded in half once along the horizontal center line of the first folded body to obtain the second folded body; and
the second folded body is folded along the horizontal center line of the second folded body to obtain the folded facial mask.

11. The facial mask component according to claim 7, wherein the facial mask sheet is configured to be folded in the following order:
the facial mask sheet is folded in half once along the vertical center line of the facial mask sheet to obtain the first folded body;
the first folded body is folded once from top to bottom along the horizontal center line on which the eye hole of the first folded body is located, so as to obtain the second folded body; and
the second folded body is folded from bottom to top along the horizontal line on which the lower edge of the mouth hole of the second folded body is located, so as to obtain the folded facial mask.

12. The facial mask component according to claim 7, wherein the facial mask component has no isolating membrane.

13. A manufacturing process of facial mask, comprising the following steps:
folding a facial mask sheet according to any one of claims 1-5 in half twice along the vertical direction and folding the same mask sheet in half once along the horizontal direction to obtain a folded facial mask, or folding the facial mask sheet in half once along the vertical direction and folding the same mask sheet in half twice along the horizontal direction to obtain the folded facial mask; or folding the facial mask sheet in half once along the vertical direction and horizontally folding the same mask sheet once along horizontal center lines on which the eye holes are located and the horizontal line on which a lower edge of the mouth hole is located respectively to obtain the folded facial mask; and
placing the folded facial mask in a packaging bag.

14. The manufacturing process of facial mask according to claim 13, wherein the folding a facial mask sheet in half twice along the vertical direction and folding the same mask sheet in half once along the horizontal direction to obtain a folded facial mask comprises the following steps:
folding the facial mask sheet in half once along the vertical center line of the facial mask sheet to obtain a first folded body;
folding the first folded body in half once along the vertical center line of the first folded body to obtain a second folded body; and
folding the second folded body along the horizontal center line of the second folded body to obtain the folded facial mask.

15. The manufacturing process of facial mask according to claim 13, wherein the folding a facial mask sheet in half twice along the vertical direction and folding the same mask sheet in half once along the horizontal direction to obtain a folded facial mask comprises the following steps:
folding the facial mask sheet in half once along the vertical center line of the facial mask sheet to obtain the first folded body;
folding the first folded body in half once along the horizontal center line of the first folded body to obtain the second folded body; and
folding the second folded body along the vertical center line of the second folded body to obtain the folded facial mask.

16. The manufacturing process of facial mask according to claim 13, wherein the folding the facial mask sheet in half once along the vertical direction and folding the same mask sheet in half twice along the horizontal direction to obtain the folded facial mask comprises the following steps:
folding the facial mask sheet in half once along the vertical center line of the facial mask sheet to obtain the first folded body;
folding the first folded body in half once along the horizontal center line of the first folded body to obtain the second folded body; and
folding the second folded body along the horizontal center line of the second folded body to obtain the folded facial mask.

17. The manufacturing process of facial mask according to claim 13, wherein the folding the facial mask sheet in half once along the vertical direction and horizontally folding the same mask sheet once along horizontal center lines of the eye holes and the horizontal line on which a lower edge of the mouth hole is located respectively to obtain the folded facial mask comprises the following steps:
folding the facial mask sheet in half once along the vertical center line of the facial mask sheet to obtain the first folded body;
folding the first folded body once from top to bottom along the horizontal center line of the eye hole of the first folded body to obtain the second folded body; and
folding the second folded body from bottom to top along the horizontal line on which the lower edge of the mouth hole of the second folded body is located to obtain the folded facial mask.

18. The manufacturing process of facial mask according to claim 13, further comprising the following steps:
filling the packaging bag with fluid; and
encapsulating the packaging bag.
